# FASCICULE DE BREVET EUROPEEN

(11) **EP 1 047 336 B1**
(45) Date de publication et mention de la délivrance du brevet: **09.06.2004**
(21) Numéro de dépôt: 99900926.9
(22) Date de dépôt: 15.01.1999
(51) Int. Cl.: A61B 5/117, G06K 9/00

(54) **DISPOSITIF D'AUTHENTIFICATION DE PERSONNE PAR SES EMPREINTES DIGITALES**
VORRICHTUNG ZUR AUTORISIERUNG VON PERSONEN MITTELS DEREN FINGERABDRÜCKEN
DEVICE FOR AUTHENTICATING A PERSON ON THE BASIS OF HIS FINGERPRINTS

(30) Priorité: 16.01.1998 FR 9800405
(43) Date de publication de la demande: 02.11.2000
(73) Titulaire: Bault, Richard V., 95370 Montigny Les Cormeilles (FR)
(72) Inventeur: Bault, Richard V., 95370 Montigny Les Cormeilles (FR)
(74) Mandataire: Kaspar, Jean-Georges
(86) Numéro de dépôt international: PCT/FR1999/000064
(87) Numéro de publication internationale: WO 1999/035964

(56) Documents cités:
- WO-A-86/06266
- WO-A-89/01674
- WO-A-94/25938
- FR-A- 2 749 955
- GB-A- 2 312 040

## Description

L'invention est relative à un dispositif d'authentification de personne par ses empreintes digitales préalablement à une autorisation d'opérations.

On connaît de nombreux systèmes d'authentification de personne basés sur la comparaison des empreintes digitales à des empreintes de référence ou des données représentatives d'empreintes de référence mémorisées à cet effet.

Les dispositifs les plus connus sont des dispositifs optiques aptes à capter l'image d'une empreinte entière, à capturer cette image pour la numériser, et à traiter les données numériques ainsi obtenues en vue de leur utilisation et de leur traitement par un moyen apte à délivrer une autorisation d'opération. Ces dispositifs optiques connus présentent l'inconvénient de nécessiter une surface de capteur optique relativement importante, supérieure ou égale à la surface de l'empreinte digitale à reconnaître.

On connut également des capteurs capacitifs, tels que le capteur commercialisé sous la dénomination "Fingertip Sensor" par la Société de Droit allemand SIEMENS AG ou le capteur commercialisé sous l'appellation "FingerLoc" (marque déposée) par la Société de Droit américain HARRIS SEMICONDUCTOR

Ces capteurs capacitifs ou électriques de type connu présentent également l'inconvénient de nécessiter une surface de capteur supérieure ou égale à celle de l'empreinte digitale à analyser.

On connaît enfin, d'après le document FR 2.749.955, un système de lecture d'empreinte digitale comportant des moyens de lecture d'empreinte digitale avec un capteur en mouvement relatif de glissement par rapport au doigt correspondant à l'empreinte digitale à analyser.

Ce capteur comportant une couche active pyroélectrique ou piézoélectrique présente une dimension inférieure à celle d'un doigt correspondant à une empreinte digitale à analyser, tandis que son autre dimension est supérieure ou égale à celle dudit doigt.

Un tel capteur est avantageux en raison de ses dimensions réduites, qui permettent d'envisager son utilisation dans des dispositifs portables de faible dimension, ou son intégration dans des dispositifs existants en vue de remplir en combinaison des fonctions additionnelles à celles de ce dispositif existant.

L'invention a pour objet un dispositif d'authentification de personne par ses empreintes digitales préalablement à une autorisation d'opération, du type comportant un boîtier portant un chemin de glissement de doigt et un capteur à couche active pyroélectrique ou piézoélectrique ayant une dimension inférieure à celle d'un doigt, disposé sur ledit chemin de glissement de doigt, dans lequel ledit chemin de glissement de doigt présente une partie bombée et ledit capteur est disposé à proximité de ladite partie bombée, de manière à régulariser et à optimiser la saisie de l'empreinte digitale d'un doigt glissant sur ledit chemin de glissement.

Selon d'autres caractéristiques de l'invention :
- le dispositif comporte un moyen de mémorisation de modèles d'empreintes de personnes bénéficiant d'une autorisation d'opération et un moyen de comparaison des modèles mémorisés dans ledit moyen de mémorisation avec une empreinte digitale saisie par le capteur, en vue de délivrer ou non une autorisation d'opération ;
- le dispositif comporte un support matériel de données contenant un logiciel apte à exécuter une mémorisation de modèles d'empreintes digitales, une modélisation de saisies numériques d'empreintes digitales et une comparaison de modèles d'empreintes digitales en vue de délivrer ou non une autorisation d'opération ;
- le dispositif comporte en outre un moyen ou un logiciel apte à effectuer une modélisation d'empreinte digitale en vue de la mémorisation du modèle d'empreinte digitale ;
- le dispositif présente une conformation de moyen de commande de la position d'un pointeur ou d'un curseur sur un écran informatique, par exemple une souris, une boule de commande ou une tablette à pression ;
- le dispositif comporte un lecteur à carte de mémoire apte à recevoir un modèle d'empreinte digitale ;
- le dispositif comporte un terminal de paiement électronique relié à ou comportant une unité informatique délivrant une autorisation d'opération ;
- le terminal de paiement électronique comporte un clavier ou autre moyen d'introduction de valeurs et/ou d'opérations alphanumériques;
- le dispositif comporte un lecteur de carte à mémoire apte à recevoir un modèle d'empreinte digitale.
- le dispositif comporte, en combinaison avec le capteur à couche active pyroélectrique ou piézoélectrique, un autre moyen, par exemple un microphone ou une caméra, permettant de mesurer une caractéristique biométrique autre que les empreintes digitales, pour réaliser une vérification biométrique multiniveaux visant à augmenter la sécurité, en vue de délivrer ou non une autorisation d'opération.

L'invention sera mieux comprise grâce à la description qui va suivre donnée à titre d'exemple non limitatif en référence aux dessins annexés dans lesquels :
- La figure 1 représente schématiquement une vue en perspective d'un premier mode de réalisation de l'invention.
- La figure 2 représente schématiquement une vue en perspective d'un deuxième mode de réalisation de l'invention
- La figure 3 représente schématiquement une vue en perspective d'un troisième mode de réalisation selon l'invention.
- La figure 4 représente schématiquement une vue en perspective d'un quatrième mode de réalisation de l'invention.
- La figure 5 représente schématiquement une vue en section par un plan médian longitudinal selon la ligne V-V de la figure 4 d'un quatrième mode de réalisation de l'invention.
- La figure 6 représente schématiquement une vue en perspective d'un cinquième mode de réalisation de l'invention.
- La figure 7 représente schématiquement une vue en perspective d'un sixième mode de réalisation de l'invention.
- La figure 8 représente schématiquement une vue en perspective d'un septième mode de réalisation de l'invention.
- La figure 9 représente schématiquement une vue en perspective d'un huitième mode de réalisation de l'invention.
- La figure 10 représente schématiquement une vue en perspective d'un neuvième mode de réalisation de l'invention.
- La figure 11 représente schématiquement une vue en perspective d'un dixième mode de réalisation de l'invention.

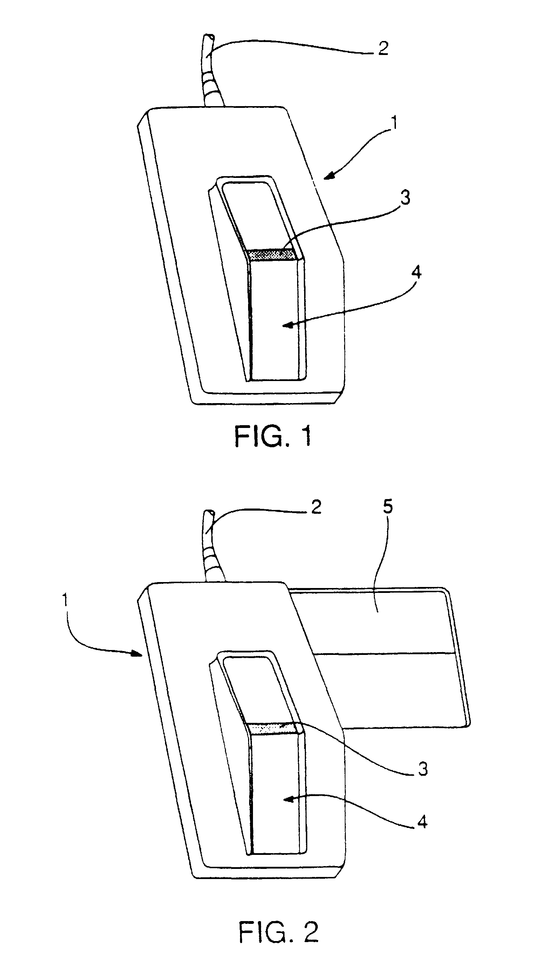

En référence à la figure 1, un premier mode de réalisation de l'invention comporte un boîtier 1 relié par un câble de raccordement 2 pour transmettre à une unité informatique (non représentée) des données en transmission série, en transmission parallèle ou à l'aide d'une transmission formant bus de communication, par exemple un bus de communication connu sous l'appellation USB (Universal Serial Bus).

Le boîtier plastique 1 contient un circuit électronique connecté à un capteur d'empreintes digitales 3 présentant une dimension inférieure à celle d'un doigt.

Le circuit électronique relié au capteur 3 et contenu dans le boîtier 1 comporte de préférence un convertisseur analogique digital, un générateur d'horloge, un adaptateur de niveau de sortie, et un connecteur de sortie permettant la transmission d'informations numériques à l'unité informatique non représentée.

Le circuit électronique comporte avantageusement un processeur spécialisé associé à une mémoire constituant une unité informatique locale.

Selon l'invention, le capteur 3 est disposé sur un chemin 4 de glissement de doigt, par exemple constitué par une partie en relief par rapport au corps du boîtier 1.

Dans l'exemple représenté, le chemin 4 de glissement est conformé en dièdre obtus et le capteur 3 est disposé au voisinage du sommet de ce dièdre obtus.

Tout autre forme de chemin de glissement, ou matérialisation de chemin de ce glissement par un marquage de couleur est comprise dans la présente invention, l'essentiel étant que le chemin de glissement de doigt présente une partie bombée, c'est-à-dire non creuse permettant de régulariser et d'optimiser la saisie de l'empreinte digitale d'un doigt glissant sur ledit chemin de glissement.

On a en effet constaté que le glissement du doigt est plus régulier et conduit à une lecture enchaînée de parties d'empreintes digitales mieux exploitable dans le cas où le chemin de glissement présente une partie bombée, c'est-à-dire non plane.

Dans ce premier mode de réalisation de l'invention, le boîtier 1 est relié à une unité informatique contenant un support matériel de données, par exemple un disque dur, une disquette, un cédérom ou moyen de mémorisation équivalent contenant un logiciel apte à être mis en oeuvre par l'unité informatique.

De préférence, ce logiciel se compose d'un pilote de communication avec le circuit électronique contenu dans le boîtier 1, d'un logiciel de signature et de reconnaissance d'empreintes digitales, d'un logiciel de gestion de la base d'empreintes permettant l'intégration des logiciels précédents dans un logiciel d'application, et permettant la communication entre l'utilisateur de l'unité informatique et l'ensemble du matériel incluant le capteur 3. Avantageusement, le logiciel de signature et de reconnaissance d'empreintes digitales vérifie, au moment de l'enregistrement d'une empreinte, que cette empreinte n'existe pas déjà dans la base de données et refuse, au moment de la vérification, une signature mathématique comportant uniquement les éléments de la signature mathématique mémorisée, de manière à éviter la réutilisation d'une signature éventuellement interceptée.

Sur la figure 2, les éléments de références identiques à celles de la figure 1 se rapportent à des éléments identiques ou fonctionnellement équivalents aux éléments de la figure 1.

Ce deuxième mode de réalisation de l'invention comporte en outre un lecteur de carte à mémoire, situé en partie inférieure du boîtier et présentant une ouverture latérale parallèle au chemin de glissement 4.

La carte à mémoire 5, par exemple une carte à puce, constitue un moyen de mémorisation de modèles d'empreintes digitales d'une personne bénéficiant d'une autorisation d'opération.

La personne à authentifier introduit la carte à mémoire 5 dans le lecteur correspondant et effectue les opérations de lecture d'empreinte digitale par passage du doigt correspondant au modèle mémorisé sur le chemin 4 de glissement.

Le logiciel précité effectue ensuite la comparaison du ou des modèles mémorisés dans la carte à mémoire 5 avec l'empreinte digitale saisie par le capteur, de manière à délivrer ou non une autorisation d'opération.

L'invention couvre également la variante selon laquelle un support matériel de données contient un logiciel de personnalisation ou de cryptage de carte à mémoire, de manière à personnaliser la structure mathématique du modèle d'empreintes digitales enregistré par la carte à mémoire.

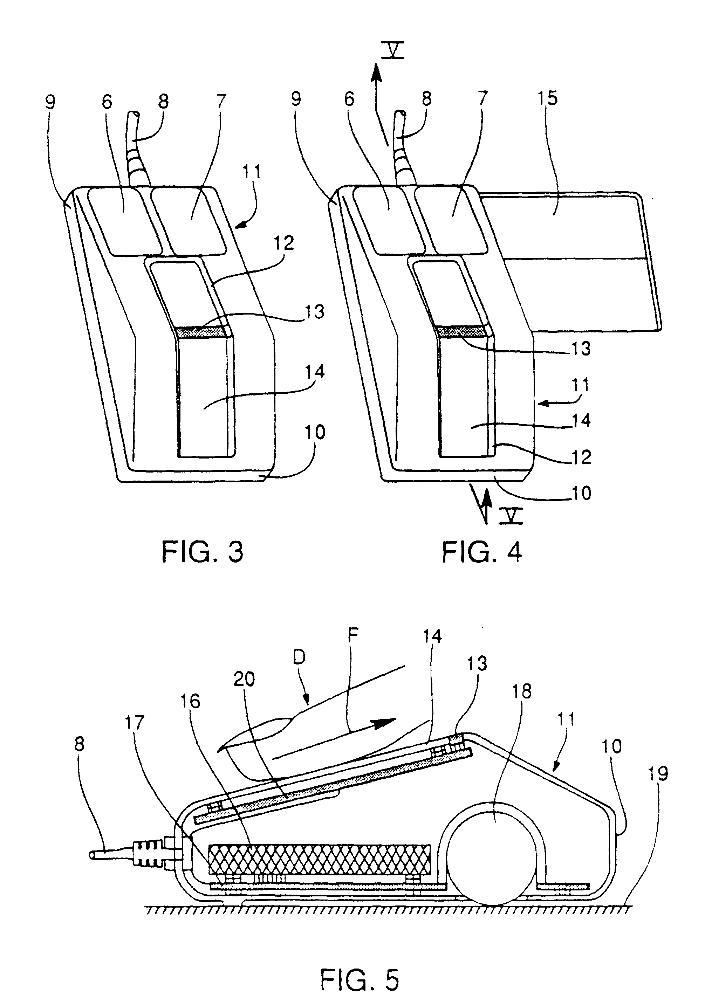

Sur la figure 3, un boîtier est conformé en moyen de commande de la position d'un pointeur ou d'un curseur sur un écran informatique. A cet effet, le boîtier présente une boule roulante sur un tapis de glissement non représenté et deux touches de commande 6 et 7 situées à l'avant du boîtier. Les boutons de commande 6 et 7 ne sont pas limités à un nombre quelconque; des commandes additionnelles telle qu'une molette ou d'autres éléments peuvent également être prévues sans sortir du cadre de la présente invention.

Un câble 8 est prévu pour relier le dispositif à une unité informatique non représentée. Un rebord 9 présentant une hauteur suffisante pour permettre le logement de la boule inférieure et des circuits électroniques s'étend périphériquement autour du dispositif.

Le rebord 9 présente une partie avant 10 apte à être retenue pour éviter le déplacement du dispositif lors de la prise d'empreintes digitales préalablement à une autorisation d'opération.

Les parties extérieures des éléments 9 et 10 précités sont intégrées ou viennent de matière avec l'ensemble 11 constituant le boîtier, réalisé par exemple en matière plastique.

Le boîtier 11 comporte un marquage ou un rebord 12 délimitant d'une part un emplacement de capteur 13 à proximité d'une partie bombée d'un chemin 14 de glissement, et entourant d'autre part la périphérie de ce chemin 14 de glissement.

Sur les figures 4 et 5, les éléments de références identiques aux références de la figure 3 désignent des éléments identiques ou fonctionnellement équivalents à ceux de la figure 3.

Le dispositif est adapté pour recevoir une carte à mémoire 15, par exemple une carte à puce apte à être lue par un lecteur 16 de carte à mémoire, en vue de comparer une empreinte digitale lue par le capteur 13 à un modèle d'empreinte digitale enregistré dans la carte à mémoire 15. Le dispositif comporte également le circuit électronique 17 associé à la fonction de commande d'un pointeur ou d'un curseur sur un écran informatique par l'intermédiaire d'un déplacement d'une boule 18 sur un tapis 19.

Le boîtier 11 contient enfin de préférence sous la partie antérieure du chemin de glissement 14 un circuit électronique 20 relié d'une part au capteur 13 et d'autre part à une unité informatique non représentée par l'intermédiaire de connexions filaires et du câble de transmission 8. Le circuit électronique 20, porte avantageusement un processeur spécialisé associé à une mémoire constituant une unité informatique locale.

Ainsi, lors du déplacement d'un doigt D dans le sens de la flèche F, des tranches d'empreinte digitale sont lues successivement par le capteur 13 et reconstituées par le logiciel contenu dans l'unité informatique après avoir été numérisées par le circuit électronique 20.

Il convient à cet effet de maintenir simplement immobile la face 10 du boîtier 11, par exemple en maintenant cette face 10 immobile par blocage contre une butée ou à l'aide d'un autre doigt de la personne sollicitant une autorisation d'opération.

Bien que décrite en référence à une souris de commande d'un pointeur ou d'un curseur sur un écran informatique, l'invention s'étend également à tout moyen de commande de la position d'un pointeur ou d'un curseur sur un écran informatique comportant un capteur disposé sur un chemin de glissement de doigt : une boule de commande, une tablette à pression, une tablette graphique, un clavier, un pavé numérique, un pointeur à loupe et réticule, un stylet à pression, un microphone de commande vocale, un casque de commande par perception du mouvement.

Egalement, l'organe de commande de la position d'un pointeur ou d'un curseur sur un écran informatique peut être reliée par liaison sans fil, par exemple par transmission infrarouge, au lieu d'être relié à l'unité informatique au moyen d'un câble 8.

L'expression "carte à mémoire" couvre non seulement la carte à puce, mais également les cartes à code-barre, à bande magnétique, à piste magnétique, les cartes aptes à être lues ou écrites à l'aide d'un laser et les cartes à code didimensionnel, notamment DATA GLYPH (marque déposée).

Le terme "boîtier" comporte également les boîtiers ou coffrets portant un autre moyen de saisie manuelle de données alphanumériques, ou les boîtiers ou coffrets comportant un moyen d'affichage, par exemple à cristaux liquides.

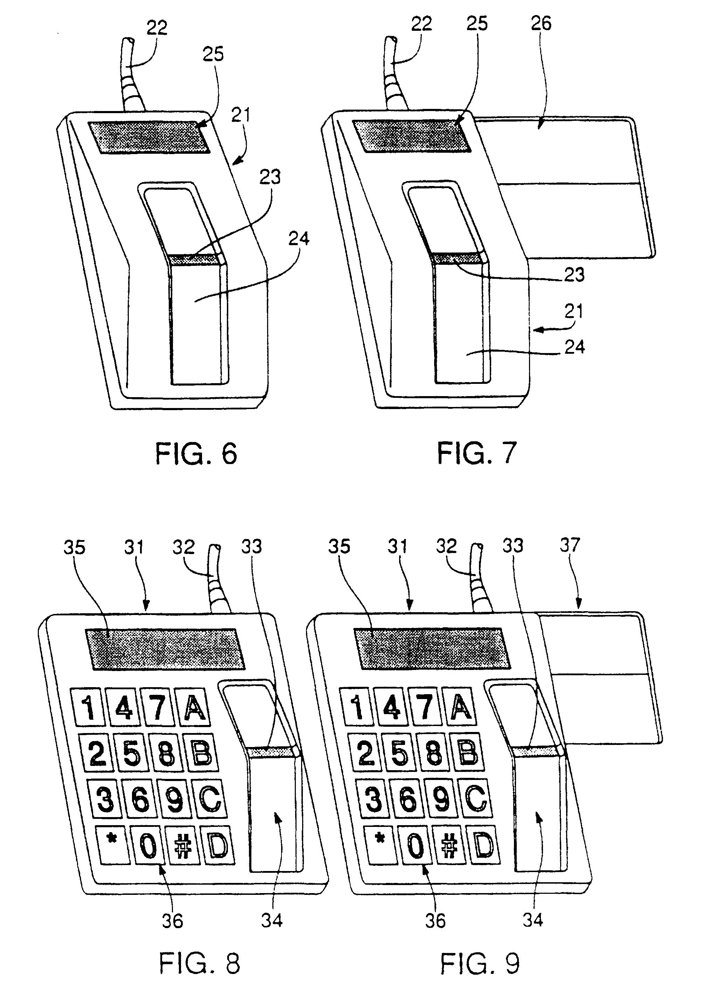

Sur la figure 6, un cinquième mode de réalisation de l'invention comporte un boîtier 21 relié par une liaison filaire 22 à une unité centrale non représentée.

Le boîtier 21 comporte un capteur 23 disposé sur un chemin de glissement 24 bombé, par exemple en forme de dièdre obtus.

Le boîtier 21 est conformé en terminal de paiement électronique destiné à saisir une empreinte digitale en vue de délivrer une autorisation d'opération de paiement. Le boîtier 21 comporte avantageusement un moyen d'affichage 25 par exemple à cristaux liquides comportant au moins une ligne d'affichage de caractères alphanumériques.

De préférence, le boîtier 21 comprend un processeur spécialisé avec une mémoire de travail, une mémoire programme, et éventuellement une mémoire flash pour la gestion de la base de données numériques représentatives de modèles d'empreintes ainsi que la tenue d'un journal des transactions de paiement électronique effectuées.

La mémoire programme éventuellement incorporée au processeur spécialisé peut être choisie dans les mémoires de types PROM ou EEPROM ou une mémoire téléchargeable de type FLASH ou EEPROM. Cette mémoire constitue un moyen matériel de support du logiciel résident. Le logiciel résident comporte une procédure d'auto-vérification du système, une initialisation et un pilotage du capteur 23 d'empreintes digitales, une saisie de tranches d'image et une reconstruction d'image entière à partir de ces tranches, une analyse et une modélisation de cette image, une comparaison du modèle d'image d'un doigt avec le modèle d'image mémorisé, éventuellement une gestion locale d'une base de données numériques représentatives de modèles d'empreintes et d'un journal de transaction.

De préférence, les éléments centralisés du logiciel sont supportés par la mémoire de l'unité informatique ou du serveur central d'autorisation de paiement : ainsi, la gestion centralisée des données représentatives des modèles mathématiques des empreintes de doigts mémorisés et la bibliothèque de primitives utilisable pour le développement de programmes d'application sont généralement mémorisés sur le serveur central.

La disposition d'un processeur spécialisé et d'un circuit électronique associé dans le boîtier 21 est avantageuse pour obtenir un fonctionnement indépendant des autres périphériques gérés par le serveur central, en faisant éventuellement appel à un bus de communication du type USB (Universal Serial Bus).

Sur la figure 7, les éléments de références identiques aux références de la figure 6 sont identiques ou fonctionnellement équivalents aux éléments de la figure 6.

Ce sixième mode de réalisation de l'invention comporte en outre en combinaison, un lecteur de carte à mémoire 26. La carte à mémoire 26 contient sous forme mémorisée des données numériques représentatives d'un modèle d'empreintes digitales de personne bénéficiant d'une autorisation d'opération.

Sur la figure 8, un terminal de paiement électronique comporte un boîtier 31 à liaison filaire 32 portant un capteur 33 d'empreintes digitales disposées sur un chemin 34 de glissement de doigt présentant une forme bombée. Le boîtier porte également un afficheur 35, par exemple à cristaux liquides et un clavier 36 ou un moyen d'introduction de valeurs ou d'opérations alphanumériques. Les données introduites à l'aide des touches alphanumériques du clavier 36 sont représentatives d'opérations financières ou de consultations ou d'introductions d'informations donnant accès à des données ou à des opérations financières.

Le terminal de paiement électronique représenté à la figure 9 est fonctionnellement équivalent au terminal de la figure 8 et comporte en outre en combinaison un lecteur de carte à mémoire. La carte à mémoire 37 contient des données numériques aptes à être utilisées pour une identification d'une personne au moyen d'une empreinte digitale saisie par le capteur 33 et comparée à un modèle d'empreints digitale mémorisé dans la carte à mémoire 37.

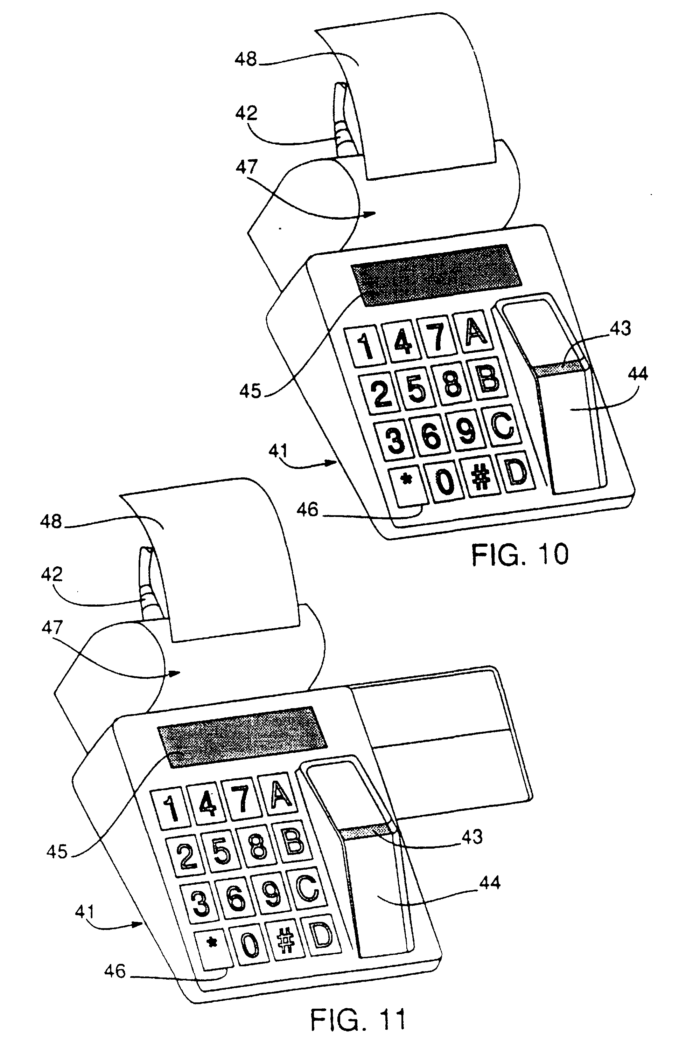

Sur la figure 10, un terminal de paiement électronique comporte un boîtier 41 relié par liaison filaire 42 à une unité centrale non représentée, un capteur 43 disposé sur un chemin de glissement de doigt 44, un afficheur 45, un clavier alphanumérique 46, une imprimante 47 commandée par le boîtier électronique 41 et accouplée avec celui-ci de manière à fournir une impression 48 représentative d'opérations ou de transactions effectuées.

Avantageusement, la feuille d'impression 48 permet de fournir, sur demande de l'unité centrale ou d'une personne autorisée, une image réelle de l'empreinte digitale de la personne sollicitant une autorisation d'opération. D'une part, cette empreinte digitale permet de mémoriser les personnes non autorisées ayant sollicité sans succès une telle autorisation, et d'autre part, l'image réelle de cette empreinte digitale est assimilable à une signature dans le cas d'une personne autorisée ne pouvant pas ou ne souhaitant pas signer, ou dans le cas de transactions importantes nécessitant un haut niveau de sécurité ainsi qu'une preuve incontestable de réalisation.

L'invention décrite en référence à des terminaux de paiement électronique couvre également en variante les coffrets ou boîtiers analogues de dimensions plus importantes, tels que les coffrets ou boîtiers de distributeurs automatiques de billets. Dans ce cas, l'invention peut être utilisée en conjonction avec un numéro d'identification personnel du type déjà utilisé pour les distributeurs automatiques de billets, de manière à procurer un niveau de sécurité supplémentaire.

Bien que les modes de réalisation des figures 6 à 11 soient décrits avec une liaison filaire, l'invention couvre également toute variante comportant une transmission sans fil, par exemple infrarouge.

Egalement, en combinaison avec tous les modes décrits de réalisation de l'invention, chaque dispositif peut comporter en combinaison avec le capteur d'empreintes digitales, au moins un capteur permettant de mesurer une caractéristique biométrique autre que les empreintes digitales, pour réaliser une vérification biométrique multi-niveaux visant à augmenter la sécurité, en vue de délivrer ou non une autorisation d'opérations : à titre d'exemple, on peut utiliser un microphone pour effectuer une reconnaissance vocale ou une caméra pour effectuer une reconnaissance visuelle de la personne sollicitant une opération d'opérations.

On va maintenant décrire un exemple de mise en oeuvre du dispositif et de son fonctionnement tels que vus par l'utilisateur.

La première étape d'utilisation du dispositif est l'enregistrement des utilisateurs. A cet effet, une autorisation de création d'un modèle est donnée avant que l'utilisateur glisse son doigt sur le chemin de glissement du capteur afin de saisir l'empreinte digitale du doigt. L'empreinte digitale du doigt est saisie par le capteur et reconstituée par le circuit électronique associé et transmise à un moyen de modélisation de saisie numérique d'empreintes digitales. Le modèle créé par ce moyen de modélisation est mémorisé dans un moyen de mémorisation de modèles d'empreintes digitales de personnes bénéficiant d'une autorisation d'opération. Cette mémorisation peut être déportée dans un support matériel de données intégré à une unité informatique, ou locale dans une mémoire de processeur spécialisé intégré au boîtier du dispositif.

De préférence, un code d'identification sera attribué à chaque utilisateur qui l'utilisera comme clé d'accès à son modèle d'empreinte digitale avant de glisser son doigt sur le capteur aux fins de vérification d'identité.

La base de données d'autorisation comportera dans ce cas à la fois les codes d'identification et les modèles d'empreintes digitales associés à ces codes d'identification.

Après cette phase d'enregistrement, l'utilisateur frappera, à chaque fois qu'il souhaite obtenir une autorisation d'opération, son code d'identification sur le clavier prévu à cet effet et glissera son doigt sur le capteur d'empreintes digitales.

Dans le cas d'une souris informatique, le clavier utilisé pour entrer le code d'identification est avantageusement le clavier de l'unité informatique.

Dans le cas d'un terminal de paiement disposant d'un clavier, le clavier d'entrée du code d'identification est de préférence le clavier du terminal de paiement. Il en est de même dans le cas d'un distributeur automatique de billets.

## Revendications

1. Dispositif d'authentification de personne par ses empreintes digitales, en vue d'authentifier ladite personne préalablement à une autorisation d'opération, du type comportant un boîtier (1, 11, 21, 31, 41) portant un chemin (4, 14, 24, 34, 44) de glissement de doigt et un capteur à couche active pyroélectrique ou piézoélectrique (3, 13, 23, 33, 43) ayant une dimension inférieure à celle d'un doigt, disposé sur ledit chemin (4, 14, 24, 34, 44) de glissement de doigt, **caractérisé en ce que** ledit chemin (4, 14, 24, 34, 44) de glissement de doigt présente une partie bombée, et **en ce que** ledit capteur (3, 13, 23, 33, 43) est disposé à proximité de ladite partie bombée, de manière à régulariser et à optimiser la saisie de l'empreinte digitale d'un doigt glissant sur ledit chemin (4, 14, 24, 34, 44) de glissement.

2. Dispositif selon la revendication 1, **caractérisé en ce que** le dispositif comporte un moyen de mémorisation de modèles d'empreintes digitales de personnes bénéficiant d'une autorisation d'opération et un moyen de comparaison des modèles mémorisés dans ledit moyen de mémorisation avec une empreinte digitale saisie par le capteur (3, 13, 23, 33, 43), en vue de délivrer ou non une autorisation d'opération.

3. Dispositif selon la revendication 2, **caractérisé en ce que** le dispositif comporte un support matériel de données contenant un logiciel apte à exécuter une mémorisation de modèles d'empreintes digitales, une modélisation des saisies numériques d'empreintes digitales et une comparaison de modèles d'empreintes digitales en vue de délivrer ou non une autorisation d'opération.

4. Dispositif selon la revendication 2 ou 3, **caractérisé en ce que** le dispositif comporte en outre un moyen ou un logiciel apte à effectuer une modélisation d'empreinte digitale en vue de la mémorisation du modèle d'empreinte digitale.

5. Dispositif de commande de la position d'un pointeur ou d'un curseur sur un écran informatique, comportant un dispositif selon l'une quelconque des revendications précédentes.

6. Dispositif selon l'une quelconque des revendications 1 à 5, **caractérisé en ce que** le dispositif comporte un lecteur de carte à mémoire (5, 15, 26) apte à recevoir un modèle d'empreinte digitale.

7. Dispositif selon l'une quelconque des revendications 1 à 3, **caractérisé en ce que** le dispositif comporte en outre un terminal (21, 31, 41) de paiement électronique comportant une unité informatique délivrant une autorisation d'opération.

8. Dispositif selon la revendication 7, **caractérisé en ce que** le terminal de paiement électronique comporte un clavier (36, 46) ou autre moyen d'introduction de valeurs et/ou d'opérations alphanumériques.

9. Dispositif selon la revendication 7 ou 8, **caractérisé en ce que** le dispositif comporte un lecteur de carte à mémoire (26, 37) apte à recevoir un modèle d'empreinte digitale.

10. Dispositif selon l'une quelconque des revendications 1 à 3, **caractérisé en ce que** le dispositif comporte en outre un terminal de paiement électronique, qui est reliable à une unité informatique délivrant une autorisation d'opération.

11. Dispositif selon l'une quelconque des revendications précédentes, **caractérisé en ce que** le dispositif comporte, en combinaison avec le capteur à couche active pyroélectrique ou piézoélectrique, un autre moyen, par exemple un microphone ou une caméra, permettant de mesurer une caractéristique biométrique autre que les empreintes digitales, pour réaliser une vérification biométrique multi-niveaux visant à augmenter la sécurité, en vue de délivrer ou non une autorisation d'opération.

## Patentansprüche

1. Vorrichtung zur Authentifizierung einer Person anhand ihrer Fingerabdrücke, um die Person vor Genehmigung eines Vorgangs zu authentifizieren, mit einem Gehäuse (1, 11, 21, 31, 41), das eine Gleitbahn (4, 14, 24, 34, 44) für den Finger und einen Sensor mit einer pyroelektrischen oder piezoelektrischen aktiven Schicht (3, 13, 23, 33, 43) trägt, der eine geringere Abmessung als ein Finger aufweist, der auf der Gleitbahn (4, 14, 24, 34, 44) für den Finger angeordnet ist, **dadurch gekennzeichnet, daß** die Gleitbahn (4, 14, 24, 34, 44) für den Finger einen gewölbten Abschnitt aufweist und der Sensor (3, 13, 23, 33, 43) nahe dem gewölbten Abschnitt angeordnet ist, um die Erfassung des Fingerabdrucks eines auf der Gleitbahn (4,14, 24, 34,44) gleitenden Fingers zu steuern und zu optimieren.

2. Vorrichtung nach Anspruch 1, **dadurch gekennzeichnet, daß** die Vorrichtung mit einer Speichereinrichtung für Fingerabdruckmodelle von Personen, die in den Genuss einer Genehmigung eines Vorgangs Kommen, und mit einer Einrichtung zum Vergleichen der in der Speichereinrichtung gespeicherten Modelle mit einem vom Sensor (3, 13, 23, 33, 43) erfaßten Fingerabdruck, um eine Genehmigung für einen Vorgang zu erteilen oder nicht, versehen ist.

3. Vorrichtung nach Anspruch 2, **dadurch gekennzeichnet, daß** die Vorrichtung einen Datenträger mit einer Software umfaßt, die dazu eingerichtet ist, eine Speicherung von Fingerabdruckmodellen, eine Modellierung der digitalen Eingaben von Fingerabdrücken und einen Vergleich von Fingerabdruckmodellen durchzuführen, um eine Genehmigung für einen Vorgang zu erteilen oder nicht.

4. Vorrichtung nach Anspruch 2 oder 3, **dadurch gekennzeichnet, daß** die Vorrichtung ferner eine Einrichtung oder eine Software umfaßt, die dazu eingerichtet ist, eine Fingerabdruck-Modellierung im Hinblick auf die Speicherung des Fingerabdruckmodells durchzuführen.

5. Vorrichtung zur Steuerung der Position eines Positionsanzeigers oder eines Cursors auf einem Bildschirm mit einer Vorrichtung nach einem der vorhergehenden Ansprüche.

6. Vorrichtung nach einem der Ansprüche 1 bis 5, **dadurch gekennzeichnet, daß** die Vorrichtung einen Speicherkartenleser (5, 15, 26) umfaßt, der dazu eingerichtet ist, ein Fingerabdruckmodell zu empfangen.

7. Vorrichtung nach einem der Ansprüche 1 bis 3, **dadurch gekennzeichnet, daß** die Vorrichtung ferner ein Terminal (21, 31, 41) zum elektronischen Bezahlen aufweist, das mit einer Rechnereinheit versehen ist, die eine Genehmigung für einen Vorgang erteilt.

8. Vorrichtung nach Anspruch 7, **dadurch gekennzeichnet, daß** das Terminal zum elektronischen Bezahlen eine Tastatur (36, 46) oder eine andere Einrichtung zur Eingabe von Werten und/oder von alphanumerischen Operationen umfaßt.

9. Vorrichtung nach Anspruch 7 oder 8, **dadurch gekennzeichnet, daß** die Vorrichtung einen Speicherkartenleser (26, 37) umfaßt, der dazu eingerichtet ist, ein Fingerabdruckmodell zu empfangen.

10. Vorrichtung nach einem der Ansprüche 1 bis 3, **dadurch gekennzeichnet, daß** die Vorrichtung ferner ein Terminal zum elektronischen Bezahlen umfaßt, das mit einer Rechnereinheit verbindbar ist, die eine Genehmigung für einen Vorgang erteilt.

11. Vorrichtung nach einem der vorhergehenden Ansprüche, **dadurch gekennzeichnet, daß** die Vorrichtung in Kombination mit dem Sensor mit einer pyroelektrischen oder piezoelektrischen aktiven Schicht eine andere Einrichtung, z.B. ein Mikrophon oder eine Kamera, umfaßt, das/die es gestattet, ein anderes biometrisches Merkmal als die Fingerabdrücke zu bestimmen, um eine mehrstufige biometrische Überprüfung, die auf eine Erhöhung der Sicherheit gerichtet ist, durchzuführen, um eine Genehmigung für einen Vorgang zu erteilen oder nicht.

## Claims

1. A device for authenticating a person on the basis of his or her fingerprints, in order to authenticate the said person before an authorisation for operation, of the type having a casing (1, 11, 21, 31, 41) carrying a strip (4, 14, 24, 34, 44) for a finger to slide on and a sensor (3, 13, 23, 33, 43) having a pyroelectric or piezoelectric active layer which is smaller in size than a finger and is arranged on the said strip (4, 14, 24, 34, 44) for a finger to slide on, **characterised in that** the said strip (4, 14, 24, 34, 44) for a finger to slide on has a bulging part, and **in that** the said sensor (3, 13, 23, 33, 43) is arranged near the said bulging part so as to regularise and optimise capture of the fingerprint of a finger sliding on the said strip (4, 14, 24, 34, 44) for sliding on.

2. A device according to Claim 1, **characterised in that** the device has a means of storing models of fingerprints of persons granted an authorisation for operation and a means of comparing models stored in the said storage means with a fingerprint captured by the sensor (3, 13, 23, 33, 43), in order to supply an authorisation for operation or not.

3. A device according to Claim 2, **characterised in that** the device has a hardware data carrier containing software suitable for executing the storage of models of fingerprints, a means of modelling the numerical data of fingerprints which is captured, and a means of comparing models of fingerprints, in order to supply an authorisation for operation or not.

4. A device according to Claim 2 or 3, **characterised in that** the device moreover has a means or software suitable for performing modelling of a fingerprint, in order to store the fingerprint model.

5. A device for controlling the position of a pointer or cursor on a computer screen, having a device according to any one of the preceding claims.

6. A device according to any one of Claims 1 to 5, **characterised in that** the device has a memory card reader (5, 15, 26) suitable for receiving a model of a fingerprint.

7. A device according to any one of Claims 1 to 3, **characterised in that** the device moreover has a terminal (21, 31, 41) for electronic payment having a computer unit supplying an authorisation for operation.

8. A device according to Claim 7, **characterised in that** the terminal for electronic payment has a keyboard (36, 46) or other means of entering alphanumeric operations and/or values.

9. A device according to Claim 7 or 8, **characterised in that** the device has a memory card reader (26, 37) suitable for receiving a model of a fingerprint.

10. A device according to any one of Claims 1 to 3, **characterised in that** the device moreover has a terminal for electronic payment which is connectable to a computer unit supplying an authorisation for operation.

11. A device according to any one of the preceding claims, **characterised in that** the device has, combined with the sensor having a pyroelectric or piezoelectric active layer, another means, for example a microphone or a camera, allowing a biometric characteristic other than the fingerprints to be measured, in order to perform a multi-level biometric check with the aim of increasing security, in order to supply an authorisation for operation or not.
